Europäisches Patentamt

European Patent Office

Office européen des brevets

(11) Publication number: **0 135 352**
**B1**

(12) **EUROPEAN PATENT SPECIFICATION**

(45) Date of publication of patent specification: 08.08.90

(51) Int. Cl.⁵: **G 01 N 33/569** // C12N7/00

(21) Application number: 84305458.6

(22) Date of filing: 10.08.84

(54) Reagent for use in detecting antibody to human T-cell leukaemia virus antigen.

(30) Priority: 22.08.83 JP 152772/83

(43) Date of publication of application:
27.03.85 Bulletin 85/13

(45) Publication of the grant of the patent:
08.08.90 Bulletin 90/32

(84) Designated Contracting States:
CH DE FR GB IT LI NL

(56) References cited:
EP-A-0 101 166
EP-A-0 105 465
WO-A-84/04327
DE-A-3 315 081
US-A-4 264 766

Journal of Clin Microbiol. Feb. 1988, p.308-312

The file contains technical information
submitted after the application was filed and
not included in this specification

(73) Proprietor: FUJIREBIO KABUSHIKI KAISHA also
trading as FUJIREBIO INC.
6-7, Shimoochiai 4-chome Shinjuku-ku
Tokyo 161 (JP)

(73) Proprietor: AJINOMOTO CO., INC.
5-8, Kyobashi 1-chome, Chuo-ku
Tokyo 104 (JP)

(72) Inventor: Tsuji, Yoshikatsu
607, Kitamikata Takatsu-ku
Kawasaki-shi Kanagawa-ken (JP)
Inventor: Ikeda, Mikio
7-25-23, Sunagawacho
Tachikawa-shi Tokyo (JP)

(74) Representative: Silverman, Warren et al
HASELTINE LAKE & CO. Hazlitt House
28 Southampton Buildings Chancery Lane
London WC2A 1AT (GB)

Courier Press, Leamington Spa, England.

# EP 0 135 352 B1

**Description**

This invention relates to a reagent for detecting an antibody to human T-cell leukaemia virus (HTLV) antigen, also known as human T-cell lymphoma virus, adult T-cell leukaemia virus and adult T-cell lymphoma virus as an indication of its different sources.

HTLV is thought to be a causative virus of cutaneous T-cell leukaemia/lymphoma. Natural antibodies to HTLV antigens are produced in sera from patients or healthy persons infected with HTLV. Therefore, since the detection of these natural antibodies can be regarded as evidence of HTLV infection, enzyme-linked immunosorbent assay (ELISA), indirect immunofluorescence assay (IF) and radioimmunoassay techniques have been developed as part of the detection of HTLV. However, these methods are complicated and time-consuming to carry out and require special equipment.

It is an object of the present invention to provide means of detecting HTLV infection easily.

According to the present invention, there is provided a reagent for use in detecting an antibody to human T-cell leukaemia virus antigen by undergoing aggregation, which reagent is constituted by gelatin particles sensitized with human T-cell leukemia virus antigen or its antigenic component.

It has now been found that gelatin particles sensitized with HTLV antigen or its antigenic component suitably aggregate in the presence of an antibody to HTLV antigen, and the specificity and the sensitivity of a detection method utilizing this haemagglutination are comparable to those of conventional tests such as the IF test.

HTLV-producing cells from any of the above-indicated sources may be used in producing the reagent of this invention. Preferred are HUT—102 and MT—2 cell lines. HUT—102 Clone B2 cell line is available, for instance, from NCI (National Cancer Institute, National Institute of Health, Bethesda, Md.). HTLV may also be isolated from new cell lines established from peripheral T-cells of cutaneous T-cell leukaemia/lymphoma patients. Cultivation of the cells may be carried out by conventional methods. HTLV is obtained from multiplied cells and from the culture supernatant.

When HTLV is prepared from the multiplied cells, the cells are collected by centrifugation, and washed with a tris-HCl buffer solution containing sodium chloride and EDTA or a phosphate buffered saline solution. The washed cells are contacted with a nonionic surfactant such as Nonidet P40, Triton X100 and Tween 20 or an anionic surfactant such as sodium dodecylsulphate, sodium laurylbenzenesulphonate and deoxy-cholate, and in this way, cells and viruses are ruptured. The treated matter is centrifuged to remove solid matter, and the supernatant obtained may be employed as the viral antigen.

On the other hand, when HTLV is prepared from the culture supernatant, highly pure HTLV can be obtained by gradient density ultracentrifugation. HTLV separated in this way is ruptured by treatment with a surfactant such as one of the above, and may be employed as the viral antigen. The HTLV fraction may also be used as such merely by losing its multiplicative ability and without rupturing of the virus.

The sensitization of HTLV antigen or its antigenic component may be carried out using a conventional method for sensitizing an antigen on carrier particles. Such methods include the method of using a coupling reagent, such as tannic acid, glutaraldehyde, bis-diazotized benzidine, tolylenediisocyanate, difluorodinitrobenzene, carbodiimides, quinones, and chromic chloride, and the adsorption method.

The gelatin particles sensitized with HTLV antigen or its antigenic component thus obtained are usually lyophilised, and when they are to be used, combined with a reconstituting solution, a diluent, a standard serum, unsensitized carrier particles and an absorbing solution.

The measurement of the antibody to HTLV antigen may be carried out by the conventional method of utilizing indirect passive haemagglutination. For example, each sample serum is placed in wells of a micro titre plate, and each dilution series is prepared. HTLV antigen-sensitized particle suspension is added to each well, and stirred. Then the mixture in each well is allowed to stand for 1 to 2 hours, and the agglutination pattern of the sensitized particles used is judged by observation. Alternatively, sample serum is mixed with the sensitized particles on a slide glass, and the agglutination pattern after 1 to 2 minutes may be judged by observation.

By using the reagent of the invention, the antibody to HTLV antigen can be detected with high sensitivity and to a high degree of accuracy compared with when using the conventional IF method. Measurement is simple in effect, and no particular equipiment is required.

The following example illustrates this invention:

## Example

(1) Preparation of HTLV Antigen

HUT—102 Clone B2 cells obtained from NCI were seeded into a RPMI—1640 culture medium containing 10% foetal bovine serum, and cultured at 37°C for 4 days while continuously passing a sterile air stream containing 5% $CO_2$ through the culture medium. 20 litres of the culture medium were centrifuged at 3,000 rpm for 10 minutes to remove cell material, and supernatant was collected. This supernatant was layered over 20 to 55% sucrose density gradient which had previously been prepared in a continous zonal rotor, and ultracentrifugation was carried out at 30,000 rpm while the supernatant was circulated.

Virus fractions were layered again over 20 to 55% continuous density gradient which had previously been prepared in a centrifuge tube and ultracentrifugation was carried out by using a swing-type rotor at 25,000 rpm for 4 hours. Virus fractions were collected, and centrifuged at 45,000 rpm for 60 minutes to precipitate virus particules which were collected. A phosphate buffered saline solution containing 0.5% Nonidet P—40® was added to these particles which were suspended therein. The suspension was stirred for 30 minutes in an ice bath, and then centrifuged at 13,000 rpm for 30 minutes. The supernatant thus obtained was employed as HTLV antigen.

(2) Preparation of Sensitized Particles

To form a phosphate buffer solution (PBS) for suspending carrier particles, 1.92 g disodium hydrogenphosphate dodecahydrate 0.291 g potassium dihydrogenphosphate 0.438 g sodium chloride, 0.25 g gum arabic, 1.5 g glycine, 0.01 g thimerosal and 1.0 ml normal rabbit serum were dissolved in purified water that total volume of the solution was 100 ml.

To form a phosphate buffer solution to serve as diluent for HTLV antigen, 1.92 g disodium hydrogenphosphate dodecahydrate 0.291 g potassium dihydrogenphosphate, 0.438 g sodium chloride, 0.25 g gum arabic, 0.15 g sodium azide and 1.0 ml normal rabbit serum were dissolved in purified water so that total volume of the solution was 100 ml.

Modified gelatin particles were prepared by the method described in Example 1 of EP—A—00 62 968. The gelatin particles were suspended in a concentration of 2.5% by weight in 0.15 M PBS (7.2) prepared for carrier particle suspension in Example 1 and additionally containing 5 ppm of tannic acid and kept at 37°C for 10 minutes. The particles were then collected by centrifuging, and washed 3 times with a saline solution. The washed particles were suspended in the PBS (7.2) in a concentration of 5%.

The HTLV antigen prepared in step (1) was diluted 50 times with phosphate buffered saline solution of pH 7.2 (0.15 M PBS (7.2) for use as an antigen diluent and this diluted antigen was mixed with an equal volume of the above suspension of tannic acid-treated particles. The mixture was kept at 37°C for 40 minutes, and in this way, the particles sensitized with HTLV antigen were obtained. The sensitized particles were washed three times with a saline solution by centrifugation, and suspended in the PBS solution prepared for carrier particles suspension described above. This suspension was lyophilised to obtain lyophilised HTLV antigen-sensitized gelatin particles.

This lyophilised HTLV antigen-sensitised gelatin particles material was made available for testing purposes with the above diluent, unsensitized carrier particles and positive serum for control as a reagent kit.

(3) Comparative Testing

By using a kit of reagents assembled as aforesaid, indirect passive haemagglutination and particle agglutination tests were carried out with various sample sera.

The results obtained are shown in the following table.

| Serum | Gelatin Particles | IF Method |
|---|---|---|
| CTL*[1]1 | 128 | + |
| 2 | 256 | + |
| 3 | 32 | + |
| 4 | 64 | + |
| 5 | 256 | + |
| 6 | 512 | + |
| 7 | 128 | + |
| 8 | 128 | + |
| 9 | 129 | + |
| 10 | 64 | + |
| 11 | 64 | + |
| 12 | 128 | + |
| 13 | 256 | + |
| 14 | 32 | + |
| 15 | 16 | + |

|  | Serum | Gelatin Particles | IF Method |
|---|---|---|---|
| RA[*2] | 1 | <16 | – |
| | 2 | " | – |
| | 3 | " | – |
| | 4 | " | – |
| | 5 | " | – |
| | 6 | " | – |
| | 7 | " | – |
| | 8 | " | – |
| SLE[*3] | 1 | <16 | – |
| | 2 | " | – |
| | 3 | " | – |
| | 4 | " | – |
| | 5 | " | – |
| | 6 | " | – |
| | 7 | " | – |

*1  Serum from cutaneous T-cell leukaemia/lymphoma patient

*2  Serum from rheumatoid arthritis patient

*3  Serum from systemic lupus erythematosus patient

Note:  Figures in the table indicate maximum serum dilutions which shows agglutination.

## Claims

1. A reagent for use in detecting an antibody to human T-cell leukaemia virus antigen by undergoing aggregation, which reagent is constituted by gelatin particles sensitized with human T-cell leukaemia virus antigen or its antigenic component.

2. A reagent as claimed in claim 1, wherein said human T-cell leukaemia virus is human T-cell lymphoma virus, adult T-cell leukaemia virus or adult T-cell lymphoma virus.

3. A reagent as claimed in claim 1, wherein said human T-cell leukaemia virus is produced by working up HTLV-producing cells.

4. A reagent as claimed in claim 3, wherein said HTLV-producing cells are obtained from HUT—102 cell line or MT—2 cell line.

5. A reagent as claimed in claim 1, wherein said human T-cell leukaemia virus antigen or its antigenic component is prepared from a culture supernatant of human T-cell leukaemia virus-producing cells.

6. A reagent as claimed in claim 5, wherein said human T-cell leukaemia virus antigen or its antigenic component is separated from supernatant obtained on effecting gradient density ultracentrifugation after rupturing of multiplied HTLV cells and viruses.

7. A reagent as claimed in any one of claims 1 to 5, when forming part of a kit of materials for use in the diagnosis of human T-cell leukaemia, which kit additionally comprises a standard serum, an unsensitised carrier, an absorbing solution, a diluent, and a reconstituting solution.

**Patentansprüche**

1. Reagenz zur Verwendung im Nachweis des Antikörpers gegen menschliche T-Zellen-Leukämie-Virusantigene mittels einer Aggregation, dadurch gekennzeichnet, daß es auf Gelatine-Teilchen aufgebaut ist, die mit menschlichem T-Zellen-Leukämie-Virusantigen oder dessen antigenischer Komponente sensibilisiert sind.

2. Reagenz gemäß Anspruch 1, bei dem das menschliche T-Zellen-Leukämie-Virus menschliches T-Zellen-Lymphoma-Virus, reifes T-Zellen-Leukämie-Virus oder reifes T-Zellen-Lymphoma-Virus ist.

3. Reagenz gemäß Anspruch 1, bei dem das menschliche T-Zellen-Leukämie-Virus erhalten ist durch Aufarbeitung von HTLV (human T-cell leukaemia virus)-produzierenden Zellen.

4. Reagenz gemäß Anspruch 3, bei dem die HTLV-produzierenden Zellen erhalten sind aus der HUT—102 Zell-Linie oder der MT—2 Zell-Linie.

5. Reagenz gemäß Anspruch 1, bei dem das menschliche T-Zellen-Leukämie-Virusantigen oder dessen antigenische Komponente hergestellt ist aus dem Kulturüberstand von Zellen, die menschliches T-Zellen-Leukämie-Virus bilden.

6. Reagenz gemäß Anspruch 1, bei dem das menschliche T-Zellen-Leukämie-Virusantigen oder dessen antigenische Komponente abgetrennt sind aus dem Überstand, der erhalten wird durch ein fortschreitendes Dichte-Ultrazentrifugieren nach dem Aufbrechen von vermehrten HTLV-Zellen und -Viren.

7. Reagenz gemäß einem der Ansprüche 1 bis 5, bei der Herstellung eines Materialsatzes zur Verwendung bei. der Diagnose von menschlicher T-Zellen-Leukämie, wobei der Satz zusätzlich ein Standardserum, einen nicht sensibilisierten Träger, eine Absorberlösung, ein Verdünnungsmittel und eine rekonstituierende Lösung enthält.

**Revendications**

1. Réactif à employer dans la détection de l'anticorps contre l'antigène du virus provoquant la leucémie des cellules-T humaines par aggrégation, lequel réactif est constitué de particules de gélatine sensibilisées à l'antigène du virus de la leucémie des cellule-T humaines ou à son déterminant antigénique.

2. Réactif selon la revendication 1, dans lequel ledit virus de la leucémie des cellules-T humaines est le virus du lymphome des cellule-T humaines, le virus de la leucémie des cellules-T adultes, ou le virus du lymphome des cellules-T adultes.

3. Réactif selon la revendication 1, dans lequel ledit virus de la leucémie des cellules-T humaines est produit en utilisant des cellules produisant du VLTH.

4. Réactif selon la revendication 3, dans lequel lesdites cellules produisant du VLTH sont obtenues depuis la lignée cellulaire HUT—102 ou la lignée cellulaire MT—2.

5. Réactif selon la revendication 1, dans lequel ledit antigène du virus de la leucémie des cellules-T humaines ou son déterminant antigénique est préparé depuis un surnageant de culture de cellules produisant le virus de la leucémie des cellules-T.

6. Réactif selon la revendication 1, dans lequel ledit antigène du virus de la leucémie des cellules-T humaines ou son déterminant antigénique est isolé du surnageant obtenu en effectuant une ultracentrifugation à gradient de densité, après rupture des cellules (VLTH) multipliées et des virus.

7. Réactif selon l'une quelconque des revendications 1 à 5, formant un élément d'une trousse de produits à utilise dans le diagnostic de la leucémie des cellules-T humaines, laquelle trousse comprend en outre un sérum standard, un support non sensibilisé, une solution absorbante, un diluant et une solution reconstituante.